(19) Europäisches Patentamt
European Patent Office
Office européen des brevets

(11) Publication number: 0 495 013 B1

# (12) EUROPEAN PATENT SPECIFICATION

(45) Date of publication of patent specification: **08.03.95**

(51) Int. Cl.[6]: **C07K 7/06**, A61K 38/00

(21) Application number: **91901058.7**

(22) Date of filing: **16.11.90**

(86) International application number:
**PCT/US90/06739**

(87) International publication number:
**WO 91/08225 (13.06.91 91/13)**

## (54) HEXAPEPTIDES WITH SULPHATE ESTER GROUPS.

(30) Priority: **27.11.89 US 441275**

(43) Date of publication of application:
**22.07.92 Bulletin 92/30**

(45) Publication of the grant of the patent:
**08.03.95 Bulletin 95/10**

(84) Designated Contracting States:
**AT BE CH DE DK ES FR GB GR IT LI LU NL SE**

(56) References cited:
**EP-A- 226 217**
**EP-A- 268 297**
**EP-A- 285 061**
**EP-A- 381 340**
**EP-A- 0 005 636**
**DE-A- 2 022 623**
**FR-A- 2 372 146**

**Technical Board of Appeal Decision T144/83**

(73) Proprietor: **FISONS CORPORATION**
**755 Jefferson Road**
**Rochester, NY 14623 (US)**

(72) Inventor: **ROSAMOND, James, Donald**
**187 Burlington Avenue**
**Rochester, NY 14617 (US)**

(74) Representative: **Wright, Robert Gordon McRae**
**FISONS plc**
**12 Derby Road**
**Loughborough**
**Leicestershire LE11 0BB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to sulphate ester containing hexapeptides possessing feeding inhibition properties and capable of stimulating the contraction of the gallbladder.

### Background of the Invention

These peptides have six amino acids. They all differ structurally from peptides known to have feeding inhibition properties (eg CCK-8, which has the structure: Asp-Tyr($SO_3H$)-Met-Gly-Trp-Met-Asp-Phe-$NH_2$, and ceruletide, which has the structure: Glp-Gln-Asp-Tyr($SO_3H$)-Thr-Gly-Trp-Met-Asp-Phe-$NH_2$. The peptides of this invention are not found in nature but rather must be synthesised. Some synthetic peptides having feeding inhibition properties, are known, for example those disclosed in European Patent Application Nos. 0226217 and 0268297. The peptides of the present invention differ from these in that the N-terminal sulphated tyrosine has been replaced by sulphated hydroxyphenylacetic acid.

### Detailed Description

According to the invention there is provided a compound of formula I:

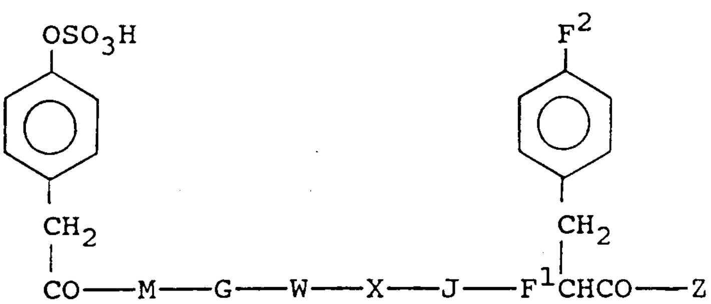

I

wherein:

M is Met, DMet, MeMet, MetO, Ahx, DAhx, MeAhx, Leu, MeLeu, Pro, Ile, MeIle, Ala or Lys;
G is Gly, DAla, Pro, Ala, $\beta$Ala or Sar;
W is Trp, MeTrp, Ala or Nal;
X is Met, MeMet, MetO, Ahx, MeAhx, Leu, MeLeu, Pro, Ile, MeIle, Ala or Lys;
J is Asp, DAsp, MeAsp, Ala or Asn;
$F^1$ is (S)-NH, (R)-NH, (S)-$R^1$N or (R)-$R^2$N;
$F^2$ is H, Cl, I, Br, F, $NO_2$, $NH_2$, $R^3$ or $OR^4$;
Z is $NH_2$, $NHR^5$ or $NR^5R^6$;
$R^1$, $R^2$, $R^3$, $R^5$ and $R^6$ are C1 to 6 alkyl; and
$R^4$ is H or C1 to 6 alkyl,
and pharmaceutically acceptable salts thereof.

According to the invention there is also provided a method of improving the bodily appearance of a mammal which comprises administering to that mammal intraperitoneally, intravenously, intramuscularly, subcutaneously or intranasally a compound of formula I, or a pharmaceutically acceptable salt thereof, until a cosmetically beneficial loss of body weight has ocurred.

We prefer compounds of formula I or pharmaceutically acceptable salts thereof, in which;

M is Met, Ahx, Leu, Ala or Ile, preferably Met, Ahx, Ala or Ile;
G is Gly or DAla, preferably Gly;
W is Trp;
X is Met, Ahx, Leu or Ile, preferably Met or Ahx;
J is Asp;
$F^1$ is (S)-NH or (S)-$R^2$N;
$F^2$ is H, $NO_2$, $R^3$ or $OR^4$, preferably H or $OR^4$;
Z is $NH_2$.

A sub-group of compounds which are preferred are those in which $R^1$, $R^2$ or $R^4$ represent methyl.

(R) and (S) refer to the absolute configurations about the adjacent methine carbon atom.

All optically active amino acids are of the L-configuration unless otherwise indicated.

$Hpa(SO_3H)$ is the formula

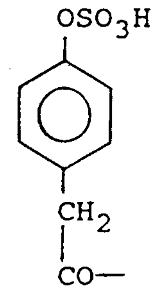

Pharmaceutically acceptable salts of compounds I include base addition salts. These include those derived from both organic and inorganic bases, for example ammonia, sodium hydroxide, barium hydroxide, tetraethylammonium hydroxide, ethylamine, diethylamine, triethylamine and the like.

For the sake of brevity, the peptides of formula I may be represented as, for example,

$Hpa(SO_3H)$-Met-Gly-Trp-Met-Asp-Phe-$NH_2$,

which stands for the compound of formula I in which M is Met, G is Gly, W is Trp, X is Met, J is Asp, $F^1$ is (S)-NH, $F^2$ is H and Z is $NH_2$.

When amino acids, peptides, protecting groups, active groups etc are represented by symbols in this specification and appended claims, usual symbols as defined by IUPAC and IUB or as used in the art are employed. Examples of symbols are given below.

| | |
|---|---|
| Abu | 2-aminobutyric acid |
| Ahx | 2-aminohexanoic acid |
| Aib | 2-aminoisobutyric acid |
| Ala | alanine |
| Arg | arginine |
| Asn | asparagine |
| Asp | aspartic acid |
| βAsp | β-aspartic acid |
| Boc | tert-butyloxycarbonyl |
| $BrCH_2$-Pam | 4-(bromomethyl)phenylacetamidomethyl |
| Cys(Me) | S-methylcysteine |
| DAla | D-alanine |
| DAhx | D,2-aminohexanoic acid |
| DAsp | D-aspartic acid |
| DMet | D-methionine |
| DPhe | D-phenylalanine |
| DPhe-$NH_2$ | D-phenylalanine amide |
| DTrp | D-tryptophan |
| DTyr | D-tyrosine |
| EtPhe | N-ethylphenylalanine |
| EtPhe-$NH_2$ | N-ethylphenylalanine amide |
| Fmoc | 9-fluorenylmethyloxycarbonyl |
| Gln | glutamine |
| Glu | glutamic acid |
| Gly | glycine |
| His | histidine |
| Hpa | 4-hydroxyphenylacetic acid |
| $Hpa(SO_3H)$ | O-sulpho-4-oxyphenylacetyl |
| Ile | isoleucine |
| Leu | leucine |
| Lys | lysine |
| MeAhx | N-methyl-2-aminohexanoic acid |
| MeAsp | N-methylaspartic acid |
| MeLeu | N-methylleucine |
| MeIle | N-methylisoleucine |

| | |
|---|---|
| MeMet | N-methylmethionine |
| MePhe | N-methylphenylalanine |
| MePhe-$NH_2$ | N-methylphenylalanine amide |
| Met | methionine |
| MetO | methionine sulphoxide |
| MeTrp | N-A-methyltryptophan |
| MeTyr | N-methyltyrosine |
| MeTyr(Me) | N,O-dimethyltyrosine |
| MeTyr(Me)-$NH_2$ | N,O-dimethyltyrosine amide |
| Mox | methoxinine |
| Nal | 3-(2-naphthyl)alanine |
| OBt | 1-benzotriazolyl ester |
| $OCH_2$-Pam | 4-oxymethylphenylacetamidomethyl |
| OSu | succinimidyloxy ester |
| OtBu | tert-butylester |
| Phe | phenylalanine |
| Phe-$NH_2$ | phenylalanine amide |
| Phe-NHEt | phenylalanine ethylamide |
| Phe-NHMe | phenylalanine methylamide |
| Phe-$N(Et)_2$ | phenylalanine diethylamide |
| Phe-$N(Me)_2$ | phenylalanine dimethylamide |
| Phe-OH | phenylalanine acid |
| Phe(4-Cl) | 3-(4-chlorophenyl)alanine |
| Phe(4-Cl)-$NH_2$ | 3-(4-chlorophenyl)alanine amide |
| Phe(4-Me) | 3-(4-methylphenyl)alanine |
| Phe(4-Me)-$NH_2$ | 3-(4-methylphenyl)alanine amide |
| Phe(4-$NO_2$) | 3-(4-nitrophenyl)alanine |
| Phe(4-$NO_2$)-$NH_2$ | 3-(4-nitrophenyl)alanine amide |
| Phe(4-$NH_2$) | 3-(4-aminophenyl)alanine |
| Phe(4-$NH_2$)-$NH_2$ | 3-(4-aminophenyl)alanine amide |
| Pro | proline |
| resin | polystyrene |
| Sar | sarcosine |
| Ser | serine |
| tBu | tert-butyl |
| Thr | threonine |
| Trp | tryptophan |
| Trp(5-F) | 5-fluorotryptophan |
| Trp(6-F) | 6-fluorotryptophan |
| Trp(Me) | 1-methyltryptophan |
| Tyr | tyrosine |
| Tyr-$NH_2$ | tyrosine amide |
| Tyr(Me) | O-methyltyrosine |
| Tyr(Me)-$NH_2$ | O-methyltyrosine amide |
| Tyr($SO_3H$) | O-sulphotyrosine |
| Val | valine |

## Preparation of Peptides

The novel sulphate ester peptides of this invention and the novel intermediates therefor may be prepared by methods well known in the art.

According to the invention there is provided a process for the preparation of compounds of formula I or a pharmaceutically acceptable salt thereof, which comprises

a) sulphating a compound of formula II:

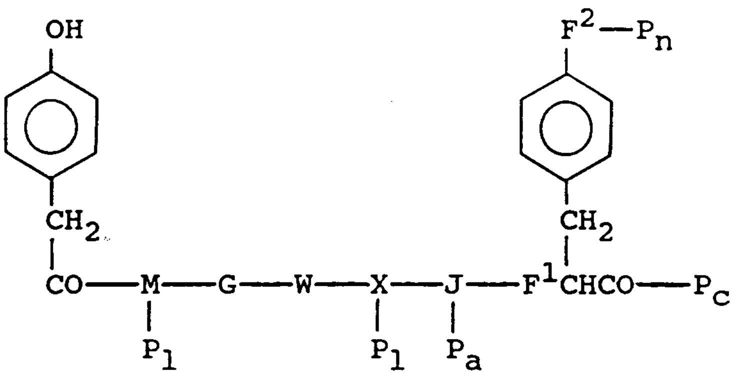

II

in which,
M, G, W, X, J, $F^1$, $F^2$ and Z are as defined above,
$P_l$ represents an amino protecting group when M or X represent Lys,
$P_a$ represents a carboxyl protecting group when J represents Asp, D-Asp or Me-Asp,
$P_n$ represents a hydroxyl protecting group or an amino protecting group when $F^2$ represents $NH_2$ or OH, and
$P_c$ represents Z or a carboxyl protecting group,
b) removing one or more protecting groups from a corresponding compound of formula III

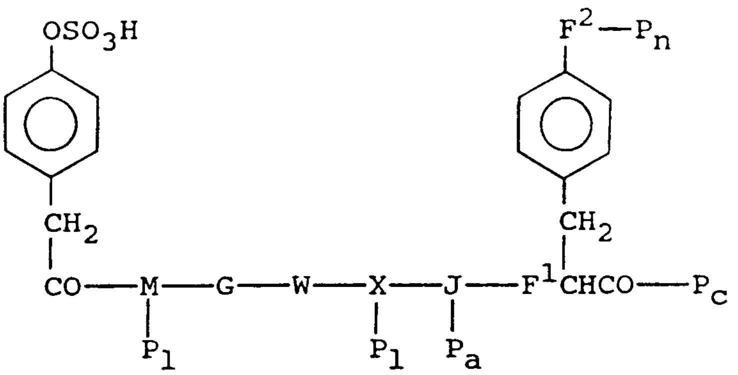

III

wherein M, G, W, X, J, $F^1$, $F^2$, $P_l$, $P_a$, $P_n$ and $P_c$ are as defined above,
provided that at least one of $P_l$, $P_a$, $P_n$ and $P_c$ represents a protecting group, and
where desired or necessary converting the resulting compound of formula I to a pharmaceutically acceptable salt thereof or vice versa.

In process a), the sulphating agent may be for example, sulphur trioxide or a complex thereof, such as sulphur trioxide pyridine. We particularly prefer to carry out the sulphation in a polar aprotic solvent, for example dimethylformamide or pyridine. The reaction is preferably carried out using an excess of sulphating agent, for example a 10 - 40 molar excess.

In process a) and b), protecting groups for peptides and methods for their removal are well known in the art, for example, T W Greene, Protective Groups in Organic Synthesis, Wiley-Interscience (1981). The choice of protecting groups and the methods employed for their removal will depend, inter alia, on the method of synthesis employed for the preparation of the peptide of formula III and the amino acids in the peptide.

Suitable amino protecting groups include, for example, benzyloxycarbonyl, which may readily be removed by hydrogenolysis or hydrogen bromide in acetic acid; t-butyloxycarbonyl, (Boc), which is removed by standing the peptide in cold trifluoroacetic acid; Fmoc, which may be removed by treatment with dilute piperidine (20% in DMF); (4-methoxybenzyl)oxycarbonyl and 2-nitrophenylsulphenyl. The Boc and Fmoc groups are particularly preferred. Suitable carboxyl protecting groups that $P_c$ may represent include, for example, methyl, tert-butyl, benzyl and 4-methoxybenzyl. We particularly prefer benzyl, which

may be readily removed by treatment with alcoholic amine or ammonia. Similar groups may be used to protect the phenol group in tyrosine, the amino group in lysine, the hydroxyl or amino groups in substituted phenylalanines and the carboxyl group in aspartate.

When the peptide is prepared using solid phase techniques, eg those in which the carboxyl end of the peptide is attached to a solid phase resin, linkage of the peptide to the resin acts as a carboxyl protecting group. Cleavage of the peptidyl-resin linkage will deprotect the carboxyl terminus of the compounds of formula II. Since the sulphate ester containing peptide end products of this invention are carboxyl terminal amides, the chemical link which connects the peptide chain to the resin must be such that its cleavage with suitable reagents readily provides amides. Due to the lability of the sulphate ester group to strong acids (for example, liquid hydrogen fluoride), the peptidyl-resin linkage may be cleavable with either weaker acids (for example, brief treatment with trifluoroacetic acid, TFA) and/or nucleophiles (for example, ammonia, amines, hydroxide, and alkoxides).

Among the suitable resin derivatives may be mentioned oxymethyl-polystyrene, 4-(oxymethylphenyl)-$(CH_2)_n$-aminomethyl-polystyrene (n = 0-3) and 4-(oxymethylphenyl)oxymethyl-polystyrene. Similarly substituted polyacrylamide resins are equally well suited as the above polystyrene based resins. The term "polystyrene" includes copolymers with minor amounts, usually 1%, of unsaturated monomers such as divinylbenzene.

4-(Oxymethylphenyl)$CH_2$CO-aminomethyl-polystyrene [herein referred to as 4-(oxymethylphenyl)-acetamidomethyl-polystyrene or $OCH_2$-Pam-resin] is particularly preferred for the generation of peptide amides. This linkage may readily be cleaved to give the peptides of formula I by reaction with methanolic solutions of ammonia, alkylamines or dialkylamines as required.

Peptides of formula III may be prepared by sulphation of a corresponding protected peptide of formula II.

Unprotected peptides of formula II may be made by deprotection of a corresponding peptide of formula II.

The novel protected peptides of formula II and the novel intermediates thereof may be prepared by methods well known to the art, for example, they may be prepared by combining individual amino acids on a solid phase resin on a step-by-step basis, or alternatively, by combining groups of amino acids on a solid phase resin to yield the desired peptidyl-resin intermediate. Such additions, as is known, are accomplished by protecting the amino group of the amino acid or group of amino acids by converting it to, for example, its tert-butyloxycarbonyl (Boc) or 9-fluorenylmethyloxycarbonyl (Fmoc) derivative, and then activating the carboxylic group of such amino acid or group of amino acids by converting it, for example, to its l-hydroxybenzotriazole (HOBt) or N-hydroxysuccinimide (HOSu) ester derivative. Such a protected-activated intermediate is then allowed to react with an amino acid resin or peptidyl-resin with a free amino group, thus extending the peptide chain to provide the peptidyl-resin of formula II.

The C-terminal amino acid of formula II may be attached to the $OCH_2$-pam-resin in several ways. (a) For example, Boc-protected N-methylphenylalanine, may be reacted with a suitable 4-(bromomethyl)-phenylacetate ester (for example, phenacyl ester) and processed further to provide Boc-MePhe(4-oxymethylphenyl)acetic acid which may be coupled to aminomethyl-polystyrene to provide Boc-MePhe-(4-oxymethylphenyl)acetamidomethylpolystyrene (Boc-MePhe-$OCH_2$-Pam-resin). (b) Alternatively, 4-(bromomethyl)phenylacetic acid may be coupled to aminomethylpolystyrene to provide 4-(bromo-methyl) phenylacetamidomethylpolystyrene ($BrCH_2$-Pam-resin) which may be reacted with the cesium salt of Boc-MePhe-OH to provide Boc-Phe-$OCH_2$-Pam-resin.

Among the suitable activating groups may be mentioned any combination of groups which causes the acid function of the amino acid to become more reactive, such as acid chlorides, mixed and symmetrical anhydrides, reaction product with carbodiimide (for example, dicyclohexylcarbodiimide, DCC), and active esters (for example, esters derived from HOBt, HOSu, 2- or 4-nitrophenol, and 2,4,5-trichlorophenol). The use of DCC and esters of HOBt and HOSu is particularly preferred from the standpoint of yield, lack of by-products, and consequent ease of purification.

An automatic peptide synthesizer may be used for the solid phase synthesis of the sulfated peptide amides of this invention. The sulphate ester containing peptides of formula I may be desalted and purified by the usual methods. For example, the product may be purified by ion-exchange chromatography with the use of Trisacryl M DEAE, DEAE-cellulose or the like, partition chromatography with the use of Sephadex® LH-20, Sephadex® G- 25 or the like, reverse phase chromatography with the use of Amberlite® XAD-2, ODS-silica gel or the like, normal phase chromatography with the use of silica gel or the like, or high-performance liquid chromatography (HPLC).

The protocol of coupling onto an aminomethyl-resin or peptidyl-$OCH_2$-Pam-resin (1 mmole of available nitrogen), deprotection, sulfation, cleavage, and product purification is set forth in Table 1.

Table 1

Protocol for Solid Phase Synthesis of Sulphated Peptide Amides

| Step | Reagent or Solvent | Purpose | Mix Time |
|---|---|---|---|
| 1 | DCM | Wash | 1 min |
| 2 | Go to step 3, 5 or 8 | ..... | ..... |
| 3 | Add filtered, pre-activated (0°C, 1 hr) mixture of protected amino acid (or protected dipeptide, 3 mmole), HOBt (4.5 mmole), and DCC (3 mmole) in 1:4 DMF/DCC | Pre-activated DCC/HOBt coupling | 2-15 hr |
| 4 | Go to step 10, 16, 21 or 26 | ..... | ..... |
| 5 | Add protected amino acid (or protected dipeptide, 3 mmole) and HOBt (4.5 mmole) in 30 ml 1:2 DMF/DCM then DCC (3 mmole) in 20 ml DCM | In situ activated DCC/HOBt coupling | 2-15 hr |
| 6 | 2-propanol | Wash | 1 min |
| 7 | Go to step 10, 16, 21 or 26 | ..... | ..... |
| 8 | Add active ester or anhydride (3 mmole)in DCM, DMF or a mixture thereof | Non DCC/HOBt activated coupling | 2-15 hr |
| 9 | Go to step 10, 16, 21 or 26 | ..... | ..... |
| 10 | DCM | Wash | 1 min |

| | | | |
|---|---|---|---|
| 11 | Treat with 49:1:50 TFA/anisole/DCM | Boc and tBu removal | 30 min |
| 12 | DCM | Wash | 1 min |
| 13 | Treat with 1:19 DIEA/DCM | Neutralise | 1 min |
| 14 | DCM | Wash | 1 min |
| 15 | Go to step 10, 16, 21 or 26 | ..... | ..... |
| 16 | DMF | Wash | 1 min |
| 17 | Treat with 1:4 piperidine/DMF | Fmoc removal | 3 min |
| 18 | Treat with 1:4 piperidine/DMF | Fmoc removal | 7 min |
| 19 | DMF | Wash | 1 min |
| 20 | Go to step 10, 16, 21 or 26 | ..... | ..... |
| 21 | DMF | Wash | 1 min |
| 22 | 1:2 pyridine/DMF | Wash | 1 min |
| 23 | Add sulphur trioxide pyridine complex (40 mmole) in 60 ml 1:2 pyridine/DMF | Sulphation | 20-24 hr |
| 24 | DMF | Wash | 1 min |
| 25 | Go to step 10, 16, 21 or 26 | ..... | ..... |
| 26 | Methanol | Wash | 1 min |
| 27 | Ammonia saturated (-20°C) methanol or 20% methanolic amine (250 ml) | Resin cleavage | 2-5 days |
| 28 | Methanol | Wash | 1 min |
| 29 | Combine and concentrate filtrates from steps 27-28 | Isolation | ..... |

30 Chromatograph residue on column(s) Purification .....
of Amberlite® XAD-2 (Rohm and Haas,
2.5 x 60 cm, methanol gradient 0.1M in ammonia),
Trisacryl M.DEAE (LKB Inc., 2.5 x 47 cm, ammonium
bicarbonate gradient), and/or P-40 ODS-3 (Whatman,
4.8 x 50 cm, methanol gradient 0.2% in ammonium
acetate)

Analogous procedures, wherein the reactions are carried out without the solid phase component (resin) are well known in the art and are well suited to large scale production. [See for example US Patent 3,892,726].

Biological Activity

The peptides of this invention have the ability to inhibit feeding activity in mammals. As a result they have utility in the prevention and treatment of obesity. Feeding inhibition activity can be demonstrated in rats as follows:

Male Sprague-Dawley rats (weighing 300-350g) are individually caged and maintained on a 12 hour light, dark cycle and trained for at least 14 days to feed during a three hour period of the dark cycle but not the 21 hours preceding that three hour period. The day of the study, rats are dosed intraperitoneally with saline (controls) or test compound (dissolved in saline; usually at a concentration of 0.3 to 300 g of test compound per kg of rat weight). Food is introduced 10 minutes after administration of saline or test compound. Test compounds are deemed to be active if the test group consumes significantly less food than the saline controls during the feeding period, which ends either 0.5 or three hours after presentation of the food.

The peptides of the invention have the ability to stimulate gallbladder contraction in mammals. Thus, they are also useful as diagnostic aids in X-ray examination of the gallbladder. The use of gallbladder contracting agents as diagnostic aids is a well established medical procedure.

The peptides of the invention have the ability to bind to cholecystokinin receptors. Distinct CCK receptors in peripheral and brain tissues have been classified as CCK-A and CCK-B receptors respectively. Differentiation between agonist and antagonist interactions at CCK receptors can also be determined by functional assays. Activation of CCK-A receptors in peripheral tissues plays an important role in the control of pancreatic secretion, gut motility and gall bladder contraction. Thus compounds with agonist activity at CCK-A receptors have utility in the treatment of obesity and motility disorders and compounds with antagonist activity at CCK-A receptors may have utility in gastrointestinal disorders such as irritable bowel syndrome, ulcers, excess pancreatic or gastric secretion , acute pancreatitis and motility disorders.

In Vitro Assays.

CCK-A Binding.

Evaluation of test compounds for their ability to bind to CCK-A receptors in rat pancreatic membranes was measured against the binding of Bolton Hunter $^{125}$I-CCK-8 and $^{3}$H-L364718 to rat pancreas according to the procedures of Chang, Lotti, Chan and Kunkel (Molecular Pharmacolgy, 30:212-216, 1986).

CCK-B Binding.

Evaluation of test compounds for their ability to bind to CCK-8-B receptors in rat cerebral cortex membranes was measured against $^{125}$I-CCK-8 according to the procedures of Chang and Lotti (Soc, Natl. Acad. Sci. Vol. 83, 4923-4926).

Functional Assay For CCK-A Agonist/Antagonist Activity.

The evaluation of test compounds for their ability to inhibit or stimulate amylase release by rat pancreatic tissue fragments (acinar cells) was measured according the procedures of Lin et al. (J of Pharm. and Exper. Therapeutics, 1986, 729-734) and Jung (Clinica Chema Acta, 1980, 100, 7-11).

The compounds of formula I, and pharmaceutically acceptable salts thereof, have the advantage that in certain pharmacological models, they are more efficacious, more potent, longer acting, more stable, particularly to enzymatic degradation, more selective, less toxic, give rise to fewer side effects, eg lack of emesis, are more readily absorbed, are quicker acting or have other advantageous effects compared to compounds of similar structure to the compounds of formula I.

According to the invention there is also provided the use of the compounds of formula I, and pharmaceutically acceptable salts thereof, in the manufacture of a medicament for use in the treatment of obesity.

According to the invention we also provide a pharmaceutical composition comprising (preferably less than 80%, and more preferably less than 50% by weight of) a compound of formula I, or a pharmaceutically acceptable salt thereof, in combination with a pharmaceutically acceptable adjuvant, diluent or carrier.

The peptides of formula I, or a pharmaceutically acceptable salt thereof, may be administered by a variety of routes, for example, intraperitoneally, intravenously, intramuscularly, subcutaneously or intranasally. The dosage of the compound of formula I will depend on several factors, including the requirements of the recipient and the particular compound employed, but will typically be in the range 0.3 µg to 3.0 mg per kg of body weight per day, either a single dose or divided among two to four doses.

The invention is illustrated, but in no way limited, by the following Examples.

An automatic peptide synthesiser was used for the solid phase synthesis of the sulphated peptide amides of the invention. The protocol of coupling onto aminomethyl- resin or peptidyl-$OCH_2$-Pam-resin (1 mmole of available nitrogen), deprotection, sulphation, cleavage and product purification is set out in table 1.

Peptide syntheses, unless otherwise stated, were initiated with 1 milliequivalent of aminomethyl-resin, where the resin was 99:1 by weight styrene:divinylbenzene copolymer. Reactions were performed at room temperature unless otherwise stated. Washing steps were performed three times with 50ml of the specified solvent unless otherwise stated.

Example 1

Boc-MePhe-(4-oxymethylphenyl)acetic Acid

To a solution of Boc-MePhe-OH (27.93g) and 4-(bromomethyl)phenylacetic acid phenacyl ester (33.32g) in acetonitrile (1000ml) was added potassium fluoride dihydrate (18.28g). The suspension was stirred overnight filtered and the filtrate evaporated to dryness. The residue, Boc-MePhe-(4-oxymethylphenyl)acetic acid phenacyl ester, was dissolved in 85% acetic acid (1200ml), treated with zinc dust (128g), and stirred for 2-4 hours.

Concentration of the filtered reaction mixture to about 400ml and dilution with 3200ml of water gave an oil which was dissolved in ethyl acetate and treated with dicyclohexylamine (DCHA) to give 41.31g of the DCHA salt of the title compound, mp 120- 122 °C.

The following compounds were prepared using essentially the same procedures:

Boc-EtPhe-(4-oxymethylphenyl)acetic acid, mp 137-141 °C (DCHA salt);
Boc-Phe(4-Cl)-(4-oxymethylphenyl)acetic acid;
Boc-Tyr(Me)-(4-oxymethylphenyl)acetic acid, mp 64-67 °C (free base);
Fmoc-Tyr(tBu)-4-oxymethylphenyl)acetic acid, 192-195 °C (free base);

Example 2

Fmoc-Met-Asp(OtBu)-OH

Fmoc-Met-OSu was prepared in situ by the reaction of Fmoc-Met-OH (14.87g) HOSu (5.52g) and dicyclohexylcarbodiimide (DCC, 8.26g) in THF (200ml) at 0 °C for 3.5 hours.

Precipitated dicyclohexylurea (DCU) was removed by filtration and the THF filtrate was added to a cold solution of H-Asp(OtBu)-OH in 220ml of 10:1 water/THF to which had been added 40ml of 1N sodium hydroxide. After stirring the reaction mixture at room temperature overnight, solid citric acid (20g) was

added along with ethyl acetate (600ml). The ethyl acetate layer was separated, washed with 10% citric acid and brine, then dried (Magnesium sulphate). Evaporation of the ethyl acetate solution gave a residue which was dissolved in ethyl acetate (200ml) and treated with DCHA (7.84ml) to precipitate 17.93g of the DCHA salt of the title compound, mp 159-162 °C.

Example 3

H-Phe-$OCH_2$-Pam-resin

Boc-Phe-(4-oxymethylphenyl)acetic acid (0.83g, 2mmole), 1-hydroxybenzotriazole (HOBt, 0.46g, 3mmole) and DCC (0.41g, 2mmole) were dissolved in 50ml of 4:1 DCM/DMF and stirred at 0 °C for 1 hour. Aminomethyl-resin (1.34g, 1mmole available nitrogen) was suspended in the filtered reaction mixture (precipitated DCU removed) and shaken for 2-15 hours. The product, Boc-Phe-$OCH_2$-Pam-resin, was isolated by filtration and treated according to table 1 (steps 10-14) to give the title compound.

H-Phe(4-Cl)-$OCH_2$-Pam-resin and H-Tyr(Me)-$OCH_2$-Pam-resin were prepared using essentially similar procedures.

Example 4

H-MePhe-$OCH_2$-Pam-resin

Boc-MePhe-(4-oxymethylphenyl)acetic acid (from 1.82g, 3 mmole of its DCHA salt) and HOBt (6.9g, 4.5mmole) in 40ml of 1:3 DMF/DCM followed by DCC (0.629, 3 mmole) in 20ml of DCM were added to aminomethyl-resin (1.34g, 1 mmole available nitrogen) to give a suspension which was shaken for 2 to 15 hours. Boc-MePhe-$OCH_2$-Pam-resin was isolated by filtration, washed with 2-propanol and DCM, and treated according to table 1 (steps 10-14) to give the title compound as free base.

H-EtPhe-$OCH_2$-Pam-resin was prepared using essentially the same procedure.

Example 5

H-Phe(4-$NO_2$)-$OCH_2$-Pam-resin

Boc-Phe(4-$NO_2$)-OH (1.39g) was dissolved in 70% methanol (100ml) and adjusted to pH 7 with the addition of 1N cesium bicarbonate. The solution was evaporated to dryness with the residue being evaporated three more times with added DMF. The resultant dried cesium salt of Boc-Phe($NO_2$)-OHwas dissolved in DMF (60ml)and shaken with $BrCH_2$-Pam-resin (1meq of Br) overnight. Boc-Phe(4-$NO_2$)-$OCH_2$-Pam-resin was isolated by filtration, washed with DCM and treated according to table 1 (steps 10-14) to give the title compound as a free base.

Example 6

H-Tyr(tBu)-$OCH_2$-Pam-resin

Fmoc-Tyr(tBu)-(4-oxymethylphenyl)acetic acid (1.82g, 3mmole), 1-hydroxybenzotriazole (0.69g, 4.5mmole) and DCC (0.62g, 3mmole) were dissolved in 50ml of 4:1 DCM/DMF and stirred at 0 °C for 1 hour. Aminomethyl-resin (1.34g, 1mmole available nitrogen) was suspended in the filtered reaction mixture (precipitated DCU removed) and shaken for 2-15 hours. Fmoc-Tyr(tBu)-$OCH_2$-Pam-resin was isolated by filtration and treated according to table 1 (steps 16-20) to give the title compound as a free base.

H-MeTyr(Me)-$OCH_2$-Pam-resin was prepared using essentially the same procedure.

Example 7

Hpa($SO_3H$)-Met-Gly-Trp-Met-Asp-Phe-$NH_2$

H-Phe-$OCH_2$-Pam-resin was sequentially coupled with Fmoc-Asp(OtBu)-OH, Fmoc-Met-OH, Fmoc-Trp-OH, Emoc-Gly-OH and Fmoc-Met-OH according to table 1 (coupling steps 5-7, followed by Fmoc removal steps 16-20) to provide H-Met-Gly-Trp-Met-Asp(OtBu)-Phe-$OCH_2$-resin which was coupled with 4-hydroxyphenylacetic acid N-hydroxysuccinimide ester (Hpa-Osu) according to table 1 (steps 10-15, steps 21-25 and then steps 26-29 with ammonia) to give the title compound which was chromatographically purified on Amberlite® XAD-2, Trisacryl M DEAE and P-40 ODS-3, sequentially, according to table 1 (step 30) to give 100mg of the ammonium salt of the title compound. Amino acid analysis following acid decomposition gave Asp 1.03 (1), Met 1.98 (2), Gly 1.02 (1) and Phe 0.98 (1). Infrared absorption spectrum showed a strong peak typical of a sulphuric acid ester at 1050 $cm^{-1}$.

Example 8

$Hpa(SO_3H)$-Ala-Gly-Trp-Met-Asp-Phe-$NH_2$

H-Phe-$OCH_2$-Pam-resin was sequentially coupled with Fmoc-Asp(OtBu)-OH, Fmoc-Met-OH, Fmoc-Trp-PH, Fmoc-Gly-OH and Fmoc-Ala-OH according to table 1 (coupling steps 5-7 followed by Fmoc removal steps 16-20) to provide H-Ala-Gly-Trp-Met-Asp(OtBu)-Phe-$OCH_2$-Pam-resin which was coupled with Hpa-OSu according to table 1 (steps 8-9) to give Hpa-Ala-Gly-Trp-Met-Asp(OtBu)-Phe-$OCH_2$-Pam-resin which was deprotected, sulphated and cleaved from the resin according to table 1 (steps 10-15, steps 21-25 and then steps 26-29 with ammonia) to give the title compound which was chromatographically purified on Amberlite® XAD-2 and P-40 ODS-3 sequentially, according to table 1 (step 30) to give 150mg of the ammonium salt of the title compound. Amino acid analysis following acid decomposition gave Asp 1.18 (1), Met 0.85 (1), Ala 0.94 (1), Gly 1.09 (1) and Phe 0.92 (1).
Infrared absorption spectrum showed a strong peak typical of a sulphuric acid ester at 1050 $cm^{-1}$.

Example 9

$Hpa(SO_3H)$-Met-Gly-Trp-Met-Asp-MePhe-$NH_2$

H-MePhe-$OCH_2$-Pam-resin was sequentially coupled with Fmoc-Met-Asp(OtBu)-OH, Fmoc-Trp-OH, Fmoc-Gly-OH and Fmoc-Met-OH according to table 1 (coupling steps 5-7 followed by Fmoc removal steps 16-20) to provide H-Met-Gly-Trp-Met-Asp(OtBu)-MePhe-$OCH_2$-Pam-resin which was coupled with Hpa-OSu according to table 1 (coupling steps 8-9) to give Hpa-Met-Gly-Trp-Met-Asp(OtBu)-MePhe-$OCH_2$-Pam-resin which was deprotected, sulphated and cleaved from the resin according to table 1 (steps 10-15, steps 21-25 and then steps 26-29 with ammonia) to give the title compound which was chromatographically purified on Amberlite® XAD-2 and P-40 ODS-3, sequentially, according to table 1 (step 30) to give 130mg of the ammonium salt of the title compound.
Amino acid analysis following acid decomposition gave Met 1.95 (2), Asp 1.00 (1), Gly 0.97 (1) and MePhe 1.08 (1). Infrared absorption spectrum showed a strong peak typical of a sulphuric acid ester at 1050 $cm^{-1}$.

Example 10

$Hpa(SO_3H)$-Ala-Gly-Trp-Met-Asp-MePhe-$NH_2$

H-MePhe-$OCH_2$-Pam-resin was sequentially coupled with Fmoc-Met-Asp(OtBu)-OH, Fmoc-Trp-OH, Fmoc-Gly-OH and Fmoc-Ala-OH according to table 1 (coupling steps 5-7 followed by Fmoc removal steps 16-20) to provide H-Ala-Gly-Trp-Met-Asp(OtBu)-MePhe-$OCH_2$-Pam-resin which was coupled with Hpa-OSu according to table 1 (coupling steps 8-9) to give Hpa-Ala-Gly-Trp-Met-Asp(OtBu)-MePhe-$OCH_2$-Pam-resin which was deprotected, sulphated and cleaved from the resin according to table 1 (steps 10-15, steps 21-25 and then steps 26-29 with ammonia) to give the title compound which was chromatographically purified on Amberlite® XAD-2 and P-40 ODS-3, sequentially, according to table 1 (step 30) to give 180mg of the ammonium salt of the title compound.
Amino acid analysis following acid decomposition gave Ala 0.97 (1), Met 0.82 (1), Gly 1.00 (1), Asp 1.10 (1) and MePhe 1.11 (1).
Infrared absorption spectrum showed a strong peak typical of a sulphuric acid ester at 1050 $cm^{-1}$.

Example 11

$Hpa(SO_3H)$-Met-DAla-Trp-Met-Asp-MePhe-$NH_2$

H-MePhe-$OCH_2$-Pam-resin was sequentially coupled with Fmoc-Met-Asp(OtBu)-OH, Fmoc-Trp-OH, Fmoc-DAla-OH and Fmoc-Met-OH according to table 1 (coupling steps 5-7 followed by Fmoc removal steps 16-20) to provide H-Met-DAla-Trp-Met-Asp(OtBu)-MePhe-$OCH_2$-Pam-resin which was coupled with Hpa-OSu according to table 1 (coupling steps 8-9) to give Hpa-Met-DAla-Trp-Met-Asp(OtBu)-MePhe-$OCH_2$-Pam-resin which was deprotected, sulphated and cleaved from the resin according to table 1 (steps 10-15, steps 21-25 and then steps 26-29 with ammonia) to give the title compound which was chromatographically purified on Amberlite® XAD-2 and P-40 ODS-3, sequentially, according to table 1 (step 30) to give 40mg of the ammonium salt of the title compound. Amino acid analysis following acid decomposition gave Asp 1.10 (1), Met 1.79 (2), Ala 0.98 (1) and MePhe 1.13 (1).
Infrared absorption spectrum showed a strong peak typical of a sulphuric acid ester at 1050 $cm^{-1}$.

Example 12

$Hpa(SO_3H)$-Met-Gly-Ala-Met-Asp-MePhe-$NH_2$

H-MePhe-$OCH_2$-Pam-resin was sequentially coupled with Fmoc-Met-Asp(OtBu)-OH, Fmoc-Ala-OH, Fmoc-Gly-OH and Fmoc-Met-OH according to table 1 (coupling steps 5-7 followed by Fmoc removal steps 16-20) to provide H-Met-Gly-Ala-Met-Asp(OtBu)-MePhe-$OCH_2$-Pam-resin which was coupled with Hpa-OSu according to table 1 (coupling steps 8-9) to give Hpa-Met-Gly-Ala-Met-Asp(OtBu)-MePhe-$OCH_2$-Pam-resin which was deprotected, sulphated and cleaved from the resin according to table 1 (steps 10-15, steps 21-25 and then steps 26-29 with ammonia) to give the title compound which was chromatographically purified on Amberlite® XAD-2 and P-40 ODS-3, sequentially, according to table 1 (step 30) to give 30mg of the ammonium salt of the title compound. Amino acid analysis following acid decomposition gave Met 1.76 (2), Asp 1.12 (1), Gly 1.07 (1) Ala 1.03 (1) and MePhe 1.02 (1).
Infrared absorption spectrum showed a strong peak typical of a sulphuric acid ester at 1050 $cm^{-1}$.

Example 13

$Hpa(SO_3H)$-Met-Gly-Trp-Ala-Asp-MePhe-$NH_2$

H-MePhe-$OCH_2$-Pam-resin was sequentially coupled with Fmoc-Ala-Asp(OtBu)-OH, Fmoc-Trp-OH, Fmoc-Gly-OH and Fmoc-Met-OH according to table 1 (coupling steps 5-7 followed by Fmoc removal steps 16-20) to provide H-Met-Gly-Trp-Ala-Asp(OtBu)-MePhe-$OCH_2$-Pam-resin which was coupled with Hpa-OSu according to table 1 (coupling steps 8-9) to give Hpa-Met-Gly-Trp-Ala-Asp(OtBu)-MePhe-$OCH_2$-Pam-resin which was deprotected, sulphated and cleaved from the resin according to table 1 (steps 10-15, steps 21-25 and then steps 26-29 with ammonia) to give the title compound which was chromatographically purified on Amberlite® XAD-2 and P-40 ODS-3, sequentially, according to table 1 (step 30) to give 140mg of the ammonium salt of the title compound.
Amino acid analysis following acid decomposition gave Ala 0.97 (1), Met 0.83 (1), Gly 0.97 (1), Asp 1.09 (1) and MePhe 1.15 (1).
Infrared absorption spectrum showed a strong peak typical of a sulphuric acid ester at 1050 $cm^{-1}$.

Example 14

$Hpa(SO_3H)$-Met-Gly-Trp-Met-Ala-MePhe-$NH_2$

H-MePhe-$OCH_2$-Pam-resin was sequentially coupled with Fmoc-Met-Ala-OH, Fmoc-Trp-OH, Fmoc-Gly-OH and Fmoc-Met-OH according to table 1 (coupling steps 5-7 followed by Fmoc removal steps 16-20) to provide H-Met-Gly-Trp-Met-Ala-MePhe-$OCH_2$-Pam-resin which was coupled with Hpa-OSu according to table 1 (coupling steps 8-9) to give Hpa-Met-Gly-Trp-Met-Ala-MePhe-$OCH_2$-Pam-resin which was deprotected, sulphated and cleaved from the resin according to table 1 (steps 10-15, steps 21-25 and then steps 26-29 with ammonia) to give the title compound which was chromatographically purified on Amberlite® XAD-2 and P-40 ODS-3, sequentially, according to table 1 (step 30) to give 170mg of the ammonium salt of the title compound. Amino acid analysis following acid decomposition gave Met 1.73 (2), Gly 0.98 (1), Ala 0.98

(1), and MePhe 1.31 (1).
Infrared absorption spectrum showed a strong peak typical of a sulphuric acid ester at 1050 $cm^{-1}$.

Example 15

$Hpa(SO_3H)$-Ala-Gly-Trp-Ala-Asp-MePhe-$NH_2$

H-MePhe-$OCH_2$-Pam-resin is sequentially coupled with Fmoc-Ala-Asp(OtBu)-OH, Fmoc-Trp-OH, Fmoc-Gly-OH and Fmoc-Ala-OH according to table 1 (coupling steps 5-7 followed by Fmoc removal steps 16-20) to provide H-Ala-Gly-Trp-Ala-Asp(OtBu)-MePhe-$OCH_2$-Pam-resin which is coupled with Hpa-OSu according to table 1 (coupling steps 8-9) to give Hpa-Ala-Gly-Trp-Ala-Asp(OtBu)-MePhe-$OCH_2$-Pam-resin which is deprotected, sulphated and cleaved from the resin according to table 1 (steps 10-15, steps 21-25 and then steps 26-29 with ammonia) to give the title compound which is chromatographically purified on Amberlite® XAD-2 and P-40 ODS-3, sequentially, according to table 1 (step 30) to give the ammonium salt of the title compound.

Example 16

$Hpa(SO_3H)$-Ahx-Gly-Trp-Ahx-Asp-MePhe-$NH_2$

H-MePhe-$OCH_2$-Pam-resin was sequentially coupled with Fmoc-Ahx-Asp(OtBu)-OH, Fmoc-Trp-OH, Fmoc-Gly-OH and Fmoc-Ahx-OH according to table 1 (coupling steps 5-7 followed by Fmoc removal steps 16-20) to provide H-Ahx-Gly-Trp-Ahx-Asp(OtBu)-MePhe-$OCH_2$-Pam-resin which was coupled with Hpa-OSu according to table 1 (coupling steps 8-9) to give Hpa-Ahx-Gly-Trp-Ahx-Asp(OtBu)-MePhe-$OCH_2$-Pam-resin which was deprotected, sulphated and cleaved from the resin according to table 1 (steps 10-15, steps 21-25 and then steps 26-29 with ammonia) to give the title compound which was chromatographically purified on Amberlite® XAD-2 and P-40 ODS-3, sequentially, according to table 1 (step 30) to give 80mg of the ammonium salt of the title compound. Amino acid analysis following acid decomposition gave Ahx 1.93 (2), Asp 1.00 (1), Gly 0.97 (1) and MePhe 1.10 (1).
Infrared absorption spectrum showed a strong peak typical of a sulphuric acid ester at 1050 $cm^{-1}$.

Example 17

$Hpa(SO_3H)$-Ile-Gly-Trp-Ile-Asp-MePhe-$NH_2$

H-MePhe-$OCH_2$-Pam-resin was sequentially coupled with Fmoc-Ile-Asp(OtBu)-OH, Fmoc-Trp-OH, Fmoc-Gly-OH and Fmoc-Ile-OH according to table 1 (coupling steps 5-7 followed by Fmoc removal steps 16-20) to provide H-Ile-Gly-Trp-Ile-Asp(OtBu)-MePhe-$OCH_2$-Pam-resin which was coupled with Hpa-OSu according to table 1 (coupling steps 8-9) to give Hpa-Ile-Gly-Trp-Ile-Asp(OtBu)-MePhe-$OCH_2$-Pam-resin which was deprotected, sulphated and cleaved from the resin according to table 1 (steps 10-15, steps 21-25 and then steps 26-29 with ammonia) to give the title compound which was chromatographically purified on Amberlite® XAD-2 and P-40 ODS-3, sequentially, according to table 1 (step 30) to give 50mg of the ammonium salt of the title compound. Amino acid analysis following acid decomposition gave Ile 1.88 (2), Asp 1.02 (1), Gly 0.98 (1) and MePhe 1.12 (1).
Infrared absorption spectrum showed a strong peak typical of a sulphuric acid ester at 1050 $cm^{-1}$.

Example 18

$Hpa(SO_3H)$-Ile-Gly-Trp-Ahx-Asp-MePhe-$NH_2$

H-MePhe-$OCH_2$-Pam-resin was sequentially coupled with Fmoc-Ahx-Asp(OtBu)-OH, Fmoc-Trp-OH, Fmoc-Gly-OH and Fmoc-Ile-OH according to table 1 (coupling steps 5-7 followed by Fmoc removal steps 16-20) to provide H-Ile-Gly-Trp-Ahx-Asp(OtBu)-MePhe-$OCH_2$-Pam-resin which was coupled with Hpa-OSu according to table 1 (coupling steps 8-9) to give Hpa-Ile-Gly-Trp-Ahx-Asp(OtBu)-MePhe-$OCH_2$-Pam-resin which was deprotected, sulphated and cleaved from the resin according to table 1 (steps 10-15, steps 21-25 and then steps 26-29 with ammonia) to give the title compound which was chromatographically purified on Amberlite® XAD-2 and P-40 ODS-3, sequentially, according to table 1 (step 30) to give 120mg of the ammonium salt of the title compound.

Amino acid analysis following acid decomposition gave Ile 1.04 (1), Trp 0.82 (1), Asp 1.04 (1), Gly 1.03 (1) Ahx 1.03 (1) and MePhe 0.86 (1).
Infrared absorption spectrum showed a strong peak typical of a sulphuric acid ester at 1050 $cm^{-1}$.

Example 19

Hpa($SO_3H$)-Met-Ala-Trp-Met-Asp-MePhe-$NH_2$

H-MePhe-$OCH_2$-Pam-resin was sequentially coupled with Fmoc-Met-Asp(OtBu)-OH, Fmoc-Trp-OH, Fmoc-Ala-OH and Fmoc-Met-OH according to table 1 (coupling steps 5-7 followed by Fmoc removal steps 16-20) to provide H-Met-Ala-Trp-Met-Asp(OtBu)-MePhe-$OCH_2$-Pam-resin which was coupled with Hpa-OSu according to table 1 (coupling steps 8-9) to give Hpa-Met-Ala-Trp-Met-Asp(OtBu)-MePhe-$OCH_2$-Pam-resin which was deprotected, sulphated and cleaved from the resin according to table 1 (steps 10-15, steps 21-25 and then steps 26-29 with ammonia) to give the title compound which was chromatographically purified on Amberlite® XAD-2 and P-40 ODS-3, sequentially, according to table 1 (step 30) to give 60mg of the ammonium salt of the title compound. Amino acid analysis following acid decomposition gave Met 1.89 (2), Asp 0.99 (1), Ala 0.99 (1) and MePhe 1.12 (1).
Infrared absorption spectrum showed a strong peak typical of a sulphuric acid ester at 1050 $cm^{-1}$.

Example 20

Hpa($SO_3H$)-Met-βAla-Trp-Met-Asp-MePhe-$NH_2$

H-MePhe-$OCH_2$-Pam-resin was sequentially coupled with Fmoc-Met-Asp(OtBu)-OH, Fmoc-Trp-OH, Fmoc-βAla-OH and Fmoc-Met-OH according to table 1 (coupling steps 5-7 followed by Fmoc removal steps 16-20) to provide H-Met-βAla-Trp-Met-Asp(OtBu)-MePhe-$OCH_2$-Pam-resin which was coupled with Hpa-OSu according to table 1 (coupling steps 8-9) to give Hpa-Met-βAla-Trp-Met-Asp(OtBu)-MePhe-$OCH_2$-Pam-resin which was deprotected, sulphated and cleaved from the resin according to table 1 (steps 10-15, steps 21-25 and then steps 26-29 with ammonia) to give the title compound which was chromatographically purified on Amberlite® XAD-2 and P-40 ODS-3, sequentially, according to table 1 (step 30) to give 41mg of the ammonium salt of the title compound. Amino acid analysis following acid decomposition gave Met 1.94 (2), Asp 1.02 (1), βAla 0.84 (1) and MePhe 1.01 (1).
Infrared absorption spectrum showed a strong peak typical of a sulphuric acid ester at 1050 $cm^{-1}$.

Example 21

Hpa($SO_3H$)-Met-Gly-Trp-Met-DAsp-MePhe-$NH_2$

As a result of racemization during the synthesis of Example 9, Hpa($SO_3H$)-Met-Gly-Trp-Met-DAsp-MePhe-$NH_2$ was isolated as a by-product during the purification of Example 9 according to Table 1 (Step 30) to give 47 mg of the ammonium salt of the title compound. Amino acid analysis following acid decomposition gave Met 1.80 (2), Gly 1.03 (1), Asp (1.06), and MePhe 1.11 (1). Infrared absorption spectrum showed a strong peak typical of a sulfuric acid ester at 1050 $cm_{-1}$. The presence of DAsp was verified by the method of Marfey (P. Marfey, Carlsberg Res. Commun., 1984, 49, 591-596).

## Claims

### Claims for the following Contracting States : AT, BE, CH, DE, DK, FR, GB, IT, LI, LU, NL, SE

1. A compound of formula I:

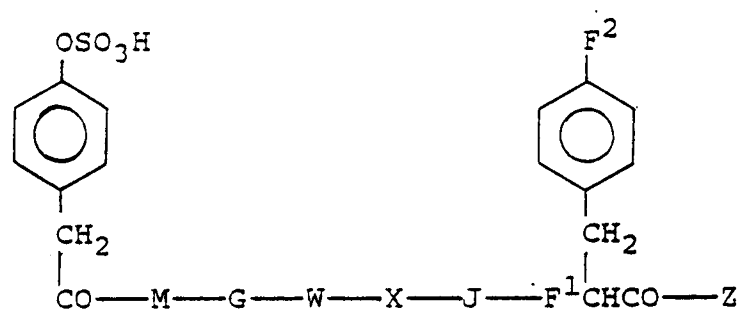

I

wherein:

M is Met, DMet, MeMet, MetO, Ahx, DAhx, MeAhx, Leu, MeLeu, Pro, Ile, MeIle, Ala or Lys;
G is Gly, DAla, Pro, Ala, $\beta$Ala or Sar;
W is Trp, MeTrp, Ala or Nal;
X is Met, MeMet, MetO, Ahx, MeAhx, Leu, MeLeu, Pro, Ile, MeIle, Ala or Lys;
J is Asp, DAsp, MeAsp, Ala or Asn;
$F^1$ is (S)-NH, (R)-NH, (S)-$R^1$N or (R)-$R^2$N;
$F^2$ is H, Cl, I, Br, F, $NO_2$, $NH_2$, $R^3$ or $OR^4$;
Z is $NH_{21}$ $NHR^5$ or $NR^5R^6$;
$R^1$, $R^2$, $R^3$, $R^5$ and $R^6$ are C1 to 6 alkyl; and
$R^4$ is H or C1 to 6 alkyl,
and pharmaceutically acceptable salts thereof, wherein Ahx represents 2-aminohexanoic acid.

2. A compound according to claim 1 wherein M is Met, Ahx, Leu, Ala or Ile.

3. A compound according to any one of the preceeding claims wherein X is Met, Ahx, Leu or Ile.

4. A compound according to any one of the preceeding claims wherein $F^1$ is (S)-NH or (S)-$R^2$N.

5. A compound according to any one of the preceeding claims wherein Z is $NH_2$.

6. A compound according to claim 1 wherein the compound of formula I is:
   Hpa($SO_3$H)-Met-Gly-Trp-Met-Asp-MePhe-$NH_2$;
   or a pharmaceutically acceptable salt thereof, wherein Hpa represents 4-hydroxyphenylacetic acid.

7. A compound according to claim 1 wherein the compound of formula I is:
   Hpa($SO_3$H)-Met-Gly-Trp-Met-Asp-Phe-$NH_2$;
   Hpa($SO_3$H)-Ala-Gly-Trp-Met-Asp-Phe-$NH_2$;
   Hpa($SO_3$H)-Ala-Gly-Trp-Met-Asp-MePhe-$NH_2$;
   Hpa($SO_3$H)-Met-DAla-Trp-Met-Asp-MePhe-$NH_2$;
   Hpa($SO_3$H)-Met-Gly-Ala-Met-Asp-MePhe-$NH_2$;
   Hpa($SO_3$H)-Met-Gly-Trp-Ala-Asp-MePhe-$NH_2$;
   Hpa($SO_3$H)-Met-Gly-Trp-Met-Ala-MePhe-$NH_2$;
   Hpa($SO_3$H)-Ala-Gly-Trp-Ala-Asp-MePhe-$NH_2$;
   Hpa($SO_3$H)-Ahx-Gly-Trp-Ahx-Asp-MePhe-$NH_2$;
   Hpa($SO_3$H)-Ile-Gly-Trp-Ile-Asp-MePhe-$NH_2$;
   Hpa($SO_3$H)-Ile-Gly-Trp-Ahx-Asp-MePhe-$NH_2$;
   Hpa($SO_3$H)-Met-Ala-Trp-Met-Asp-MePhe-$NH_2$;
   Hpa($SO_3$H)-Met-$\beta$Ala-Trp-Met-Asp-MePhe-$NH_2$;
   Hpa($SO_3$H)-Met-Gly-Trp-Met-DAsp-MePhe-$NH_2$;
   or a pharmaceutically acceptable salt thereof.

**8.** A pharmaceutical composition comprising a compound of formula I, as defined in claim 1, or a pharmaceutically acceptable salt thereof, in admixture with a pharmaceutically acceptable carrier.

**9.** The use of a compound of formula I, as defined in claim 1, or a pharmaceutically acceptable salt thereof, in the manufacture of a medicament for the treatment or prevention of obesity.

**10.** A process for the preparation of compounds of formula I, as defined in claim 1, which comprises,

a) sulphating a compound of formula II:

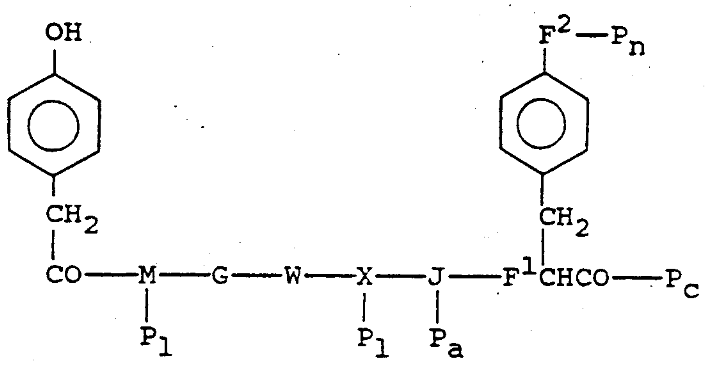

II

in which,

M, G, W, X, J, $F^1$, $F^2$ and Z are as defined above ,

$P_l$ represents an amino protecting group when M or X represent Lys,

$P_a$ represents a carboxyl protecting group when J represents Asp, D-Asp or Me-Asp,

$P_n$ represents a hydroxyl protecting group or an amino protecting group when $F^2$ represents $NH_2$ or OH, and

$P_c$ represents Z or a carboxyl protecting group,

b) removing one or more protecting groups from a corresponding compound of formula III,

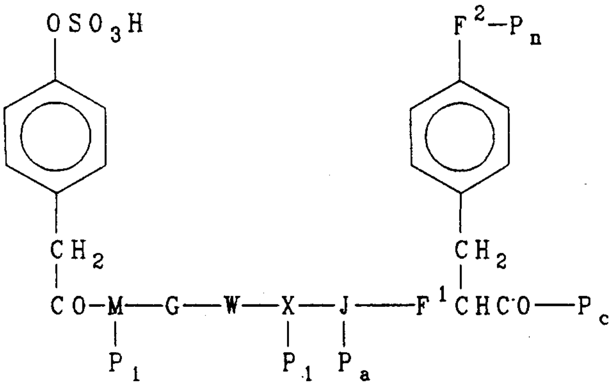

III

wherein M, G, W, X, J, $F^1$, $F^2$, $P_l$, $P_a$, $P_n$ and $P_c$ are as defined above,

provided that at least one of $P_l$, $P_a$, $P_n$ and $P_c$ represents a protecting group, and

where desired or necessary converting the resulting compound of formula I to a pharmaceutically acceptable salt thereof or *vice versa*.

**11.** A compound of formula I, as defined in claim 1, or a pharmaceutically acceptable salt thereof, for use as a pharmaceutical.

**12.** A method of improving the bodily appearance of a mammal which comprises administering to that mammal intraperitoneally, intravenously, intramuscularly, subcutaneously or intranasally a compound of formula I as defined in claim 1, or a pharmaceutically acceptable salt thereof, until a cosmetically beneficial loss of body weight has occurred.

**Claims for the following Contracting States : ES, GR**

**1.** A process for the preparation of a compound of formula I,

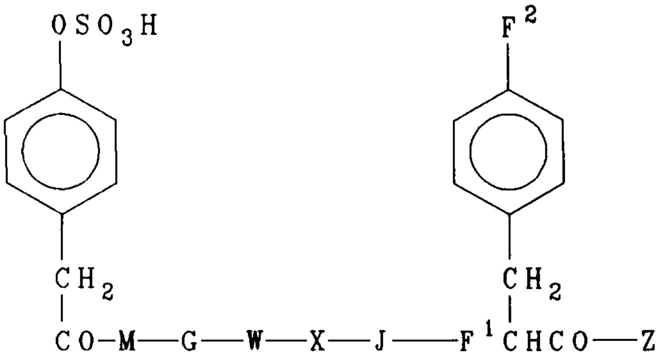

I

wherein
M is Met, DMet, MeMet, MetO, Ahx, DAhx, MeAhx, Leu, MeLeu, Pro, Ile, MeIle, Ala or Lys;
G is Gly, DAla, Pro, Ala, $\beta$Ala or Sar;
W is Trp, MeTrp, Ala or Nal;
X is Met, MeMet, MetO, Ahx, MeAhx, Leu, MeLeu, Pro, Ile, MeIle, Ala or Lys;
J is Asp, DAsp, MeAsp, Ala or Asn;
$F^1$ is (S)-NH, (R)-NH, (S)-$R^1$N or (R)-$R^2$N;
$F^2$ is H, Cl, I, Br, F, $NO_2$, $NH_2$, $R^3$ or $OR^4$;
Z is $NH_2$, $NHR^5$ or $NR^5R^6$;
$R^1$, $R^2$, $R^3$, $R^5$ and $R^6$ are C1 to 6 alkyl; and
$R^4$ is H or C1 to 6 alkyl;
wherein Ahx represents 2-aminohexanoic acid;
or a pharmaceutically acceptable salt thereof;
which comprises:
a) sulphating a compound of formula II,

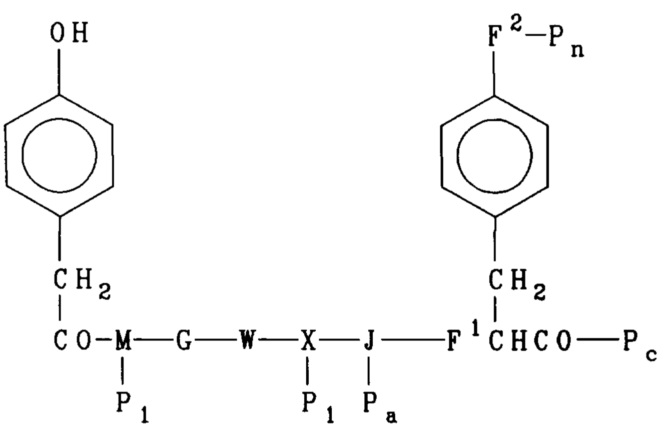

I I

in which
M, G, W, X, J, $F^1$, $F^2$ and Z are as defined above,
$P_l$ represents an amino protecting group when M or X represent Lys,
$P_a$ represents a carboxyl protecting group when J represents Asp, D-Asp or Me-Asp,
$P_n$ represents a hydroxyl protecting group or an amino protecting group when $F^2$ represents $NH_2$ or OH, and
$P_c$ represents Z or a carboxyl protecting group,

b) removing one or more protecting groups from a corresponding compound of formula III,

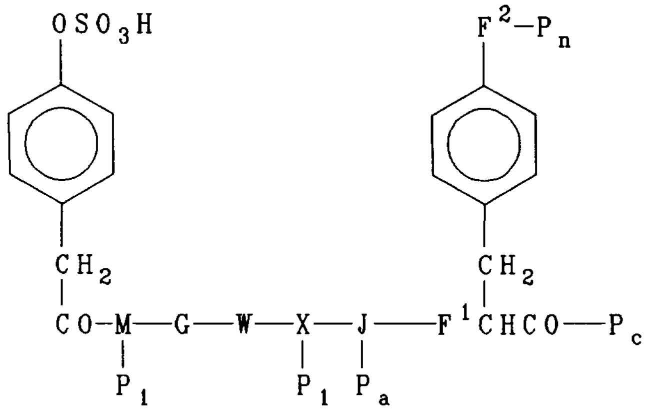

III

wherein M, G, W, X, J, $F^1$, $F^2$, $P_l$, $P_a$, $P_n$ and $P_c$ are as defined above, provided that at least one of $P_l$, $P_a$, $P_n$ and $P_c$ represents a protecting group, and where desired or necessary converting the resulting compound of formula I to a pharmaceutically acceptable salt thereof or *vice versa*.

**2.** The process according to claim 1 wherein M is Met, Ahx, Leu, Ala or Ile.

**3.** The process according to any one of the preceding claims wherein X is Met, Ahx, Leu or Ile.

**4.** The process according to any one of the preceding claims wherein $F^1$ is (S)-NH or (S)-$R^2N$.

**5.** The process according to any one of the preceding claims wherein Z is $NH_2$.

**6.** The process according to claim 1 wherein the compound of formula I is:
Hpa($SO_3H$)-Met-Gly-Trp-Met-Asp-MePhe-$NH_2$;
wherein Hpa represents 4-hydroxyphenylacetic acid;
or a pharmaceutically acceptable salt thereof.

**7.** The process according to claim 1 wherein the compound of formula I is:
Hpa($SO_3H$)-Met-Gly-Trp-Met-Asp-Phe-$NH_2$;
Hpa($SO_3H$)-Ala-Gly-Trp-Met-Asp-Phe-$NH_2$;
Hpa($SO_3H$)-Ala-Gly-Trp-Met-Asp-MePhe-$NH_2$;
Hpa($SO_3H$)-Met-DAla-Trp-Met-Asp-MePhe-$NH_2$;
Hpa($SO_3H$)-Met-Gly-Ala-Met-Asp-MePhe-$NH_2$;
Hpa($SO_3H$)-Met-Gly-Trp-Ala-Asp-MePhe-$NH_2$;
Hpa($SO_3H$)-Met-Gly-Trp-Met-Ala-MePhe-$NH_2$;
Hpa($SO_3H$)-Ala-Gly-Trp-Ala-Asp-MePhe-$NH_2$;
Hpa($SO_3H$)-Ahx-Gly-Trp-Ahx-Asp-MePhe-$NH_2$;
Hpa($SO_3H$)-Ile-Gly-Trp-Ile-Asp-MePhe-$NH_2$;
Hpa($SO_3H$)-Ile-Gly-Trp-Ahx-Asp-MePhe-$NH_2$;
Hpa($SO_3H$)-Met-Ala-Trp-Met-Asp-MePhe-$NH_2$;
Hpa($SO_3H$)-Met-$\beta$Ala-Trp-Met-Asp-MePhe-$NH_2$;
Hpa($SO_3H$)-Met-Gly-Trp-Met-DAsp-MePhe-$NH_2$;
or a pharmaceutically acceptable salt thereof.

**8.** A method of improving the bodily appearance of a mammal which comprises administering to that mammal intraperitoneally, intravenously, intramuscularly, subcutaneously or intranasally a compound of formula I as defined in claim 1, or a pharmaceutically acceptable salt thereof, until a cosmetically beneficial loss of body weight has occurred.

## Patentansprüche

## Patentansprüche für folgende Vertragsstaaten : AT, BE, CH, DE, DK, FR, GB, IT, LI, LU, NL, SE

1. Verbindung der Formel (I):

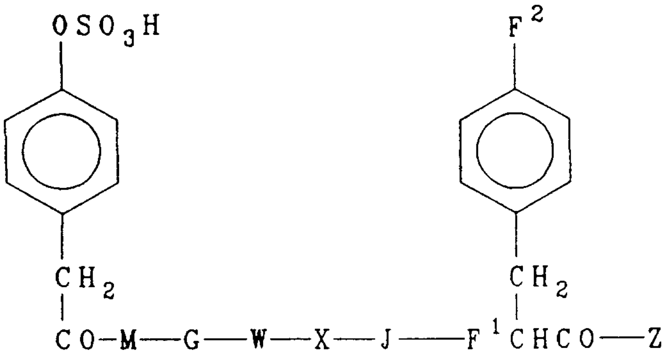

(I),

worin M Met, DMet, MeMet, MetO, Ahx, DAhx, MeAhx, Leu, MeLeu, pro, Ile, MeIle, Ala oder Lys bedeutet; G Gly, DAla, Pro, Ala, βAla oder Sar darstellt; W Trp, MeTrp, Ala oder Nal ist; X Met, MeMet, MetO, Ahx, MeAhx, Leu, MeLeu, Pro, Ile, MeIle, Ala oder Lys bedeutet; J Asp, DAsp, MeAsp, Ala oder Asn darstellt; $F^1$ (S)-NH, (R)-NH, (S)-$R^1$N oder (R)-$R^2$N ist; $F^2$ H, Cl, J, Br, F, $NO_2$, $NH_2$, $R^3$ oder $OR^4$ bedeutet; Z $NH_2$, $NHR^5$ oder $NR^5R^6$ darstellt; $R^1$, $R^2$, $R^3$, $R^5$ und $R^6$ $C_1$-$C_6$-Alkyl sind; und $R^4$ H oder $C_1$-$C_6$-Alkyl ist; und pharmazeutisch annehmbare Salze hievon, wobei Ahx 2-Aminohexansäure bedeutet.

2. Verbindung nach Anspruch 1, worin M Met, Ahx, Leu, Ala oder Ile bedeutet.

3. Verbindung nach einem der vorhergehenden Ansprüche, worin X Met, Ahx, Leu oder Ile bedeutet.

4. Verbindung nach einem der vorhergehenden Ansprüche, worin $F^1$ (S)-NH oder (S)-$R^2$N ist.

5. Verbindung nach einem der vorhergehenden Ansprüche, worin Z $NH_2$ darstellt.

6. Verbindung nach Anspruch 1, wobei die Verbindung der Formel (I) Hpa($SO_3$H)-Met-Gly-Trp-Met-Asp-MePhe-$NH_2$ oder ein pharmazeutisch annehmbares Salz hievon, wobei Hpa 4-Hydroxyphenylessigsäure bedeutet, ist.

7. Verbindung nach Anspruch 1, wobei die Verbindung der Formel (I)
   Hpa($SO_3$H)-Met-Gly-Trp-Met-Asp-Phe-$NH_2$;
   Hpa($SO_3$H)-Ala-Gly-Trp-Met-Asp-Phe-$NH_2$;
   Hpa($SO_3$H)-Ala-Gly-Trp-Met-Asp-MePhe-$NH_2$;
   Hpa($SO_3$H)-Met-DAla-Trp-Met-Asp-MePhe-$NH_2$;
   Hpa($SO_3$H)-Met-Gly-Ala-Met-Asp-MePhe-$NH_2$;
   Hpa($SO_3$H)-Met-Gly-Trp-Ala-Asp-MePhe-$NH_2$;
   Hpa($SO_3$H)-Met-Gly-Trp-Met-Ala-MePhe-$NH_2$;
   Hpa($SO_3$H)-Ala-Gly-Trp-Ala-Asp-MePhe-$NH_2$;
   Hpa($SO_3$H)-Ahx-Gly-Trp-Ahx-Asp-MePhe-$NH_2$;
   Hpa($SO_3$H)-Ile-Gly-Trp-Ile-Asp-MePhe-$NH_2$;
   Hpa($SO_3$H)-Ile-Gly-Trp-Ahx-Asp-MePhe-$NH_2$;
   Hpa($SO_3$H)-Met-Ala-Trp-Met-Asp-MePhe-$NH_2$;
   Hpa($SO_3$H)-Met-βAla-Trp-Met-Asp-MePhe-$NH_2$;
   Hpa($SO_3$H)-Met-Gly-Trp-Met-DAsp-MePhe-$NH_2$;
   oder ein pharmazeutisch annehmbares Salz hievon ist.

8. Pharmazeutische Zusammensetzung, welche eine Verbindung der Formel (I), wie in Anspruch 1 definiert, oder ein pharmazeutisch annehmbares Salz hievon, in Mischung mit einem pharmazeutisch

annehmbaren Träger umfaßt.

**9.** Verwendung einer Verbindung der Formel (I), wie in Anspruch 1 definiert, oder eines pharmazeutisch annehmbares Salzes hievon, bei der Herstellung eines Medikaments zur Behandlung oder Prävention von Obesität.

**10.** Verfahren zur Herstellung von Verbindungen der Formel (I), wie in Anspruch 1 definiert, welches umfaßt:

a) Sulfatieren einer Verbindung der Formel (II):

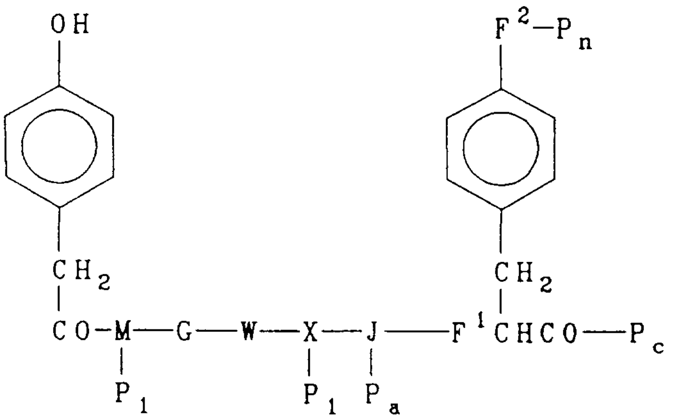

(II),

worin M, G, W, X, J, $F^1$, $F^2$ und Z wie oben definiert sind, $P_1$ eine Amino-Schutzgruppe bedeutet, wenn M oder X Lys darstellt, $P_a$ eine Carbonyl-Schutzgruppe ist, wenn J Asp, DAsp oder MeAsp darstellt, $P_n$ eine Hydroxyl-Schutzgruppe oder eine Amino-Schutzgruppe bedeutet, wenn $F^2$ $NH_2$ oder OH ist, und $P_c$ Z oder eine Carbonyl-Schutzgruppe darstellt,

b) Entfernen einer oder mehrerer Schutzgruppen aus einer entsprechenden Verbindung der Formel (III):

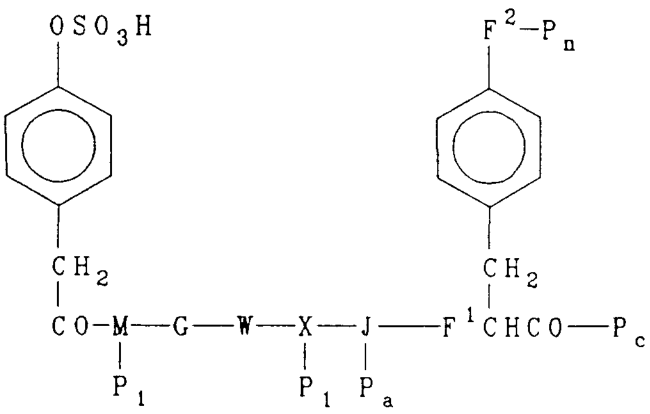

(III),

worin M, G, W, X, J, $F^1$, $F^2$, $P_1$, $P_a$, $P_n$ und $P_c$ wie oben definiert sind, mit der Maßgabe, daß zumindest eine von $P_1$, $P_a$, $P_n$ und $P_c$ eine Schutzgrppe darstellt, und, wo erwünscht oder notwendig, Überführen der erhaltenen Verbindung der Formel (I) in ein pharmazeutisch annehmbares Salz hievon oder umgekehrt.

**11.** Verbindung der Formel (I), wie in Anspruch 1 definiert, oder ein pharmazeutisch annehmbares Salz hievon, zur Verwendung als Pharmazeutikum.

**12.** Verfahren zur Verbesserung des körperlichen Aussehens eines Säugers, welches das intraperitoneale, intravenöse, intramuskuläre, subkutane oder intranasale Verabreichen einer Verbindung der Formel (I), wie in Anspruch 1 definiert, oder eines pharmazeutisch annehmbaren Salzes hievon, an diesen Säuger

umfaßt, bis ein kosmetisch vorteilhafter Körpergewichtsverlust eingetreten ist.

**Patentansprüche für folgende Vertragsstaaten : ES, GR**

1. Verfahren zur Herstellung einer Verbindung der Formel (I):

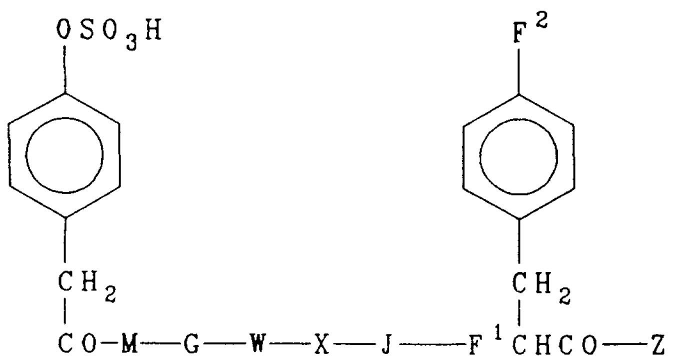

(I),

worin M Met, DMet, MeMet, MetO, Ahx, DAhx, MeAhx, Leu, MeLeu, Pro, Ile, MeIle, Ala oder Lys bedeutet; G Gly, DAla, Pro, Ala, βAla oder Sar darstellt; W Trp, MeTrp, Ala oder Nal ist; X Met, MeMet, MetO, Ahx, MeAhx, Leu, MeLeu, Pro, Ile, MeIle, Ala oder Lys bedeutet; J Asp, DAsp, MeAsp, Ala oder Asn darstellt; $F^1$ (S)-NH, (R)-NH, (S)-$R^1$N oder (R)-$R^2$N ist; $F^2$ H, Cl, J, Br, F, $NO_2$, $NH_2$, $R^3$ oder $OR^4$ bedeutet; Z $NH_2$, $NHR^5$ oder $NR^5R^6$ darstellt; $R^1$, $R^2$, $R^3$, $R^5$ und $R^6$ $C_1$-$C_6$-Alkyl sind; und $R^4$ H oder $C_1$-$C_6$-Alkyl ist; wobei Ahx 2-Aminohexansäure bedeutet, oder eines pharmazeutisch annehmbaren Salzes hievon, welches Verfahren umfaßt:

a) Sulfatieren einer Verbindung der Formel (II):

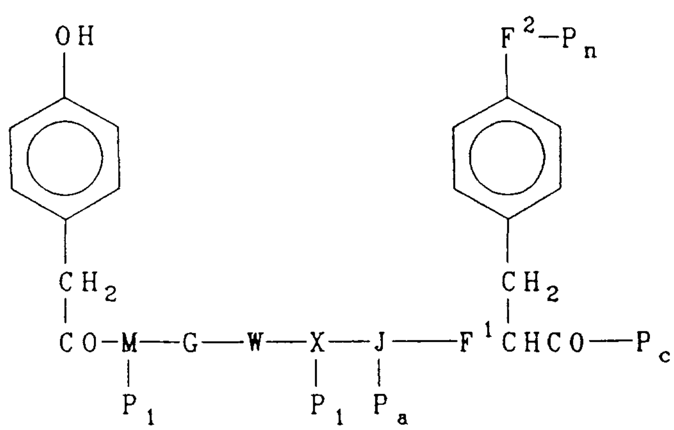

(II),

worin M, G, W, X, J, $F^1$, $F^2$ und Z wie oben definiert sind, $P_1$ eine Amino-Schutzgruppe bedeutet, wenn M oder X Lys darstellt, $P_a$ eine Carbonyl-Schutzgruppe ist, wenn J Asp, DAsp oder MeAsp darstellt, $P_n$ eine Hydroxyl-Schutzgruppe oder eine Amino-Schutzgruppe bedeutet, wenn $F^2$ $NH_2$ oder OH ist, und $P_c$ Z oder eine Carbonyl-Schutzgruppe darstellt,

b) Entfernen einer oder mehrerer Schutzgruppen aus einer entsprechenden Verbindung der Formel (III):

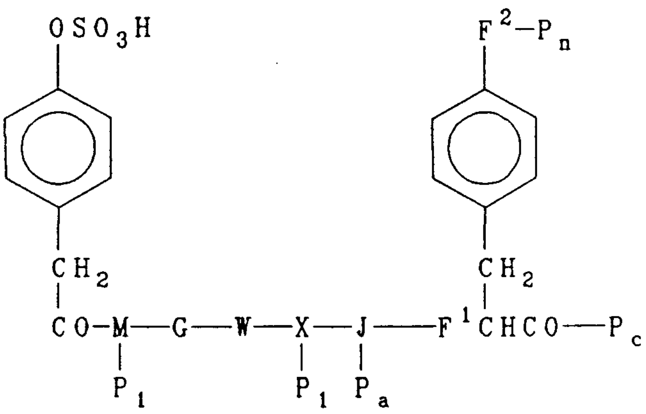

(III),

worin M, G, W, X, J, $F^1$, $F^2$, $P_1$, $P_a$, $P_n$ und $P_c$ wie oben definiert sind, mit der Maßgabe, daß zumindest eine von $P_1$, $P_a$, $P_n$ und $P_c$ eine Schutzgrppe darstellt, und, wo erwünscht oder notwendig, Überführen der erhaltenen Verbindung der Formel (I) in ein pharmazeutisch annehmbares Salz hievon oder umgekehrt.

**2.** Verfahren nach Anspruch 1, wobei M Met, Ahx, Leu, Ala oder Ile bedeutet.

**3.** Verfahren nach einem der vorhergehenden Ansprüche, wobei X Met, Ahx, Leu oder Ile bedeutet.

**4.** Verfahren nach einem der vorhergehenden Ansprüche, wobei $F^1$ (S)-NH oder (S)-$R^2N$ ist.

**5.** Verfahren nach einem der vorhergehenden Ansprüche, wobei Z $NH_2$ darstellt.

**6.** Verfahren nach Anspruch 1, bei welchem die Verbindung der Formel (I) Hpa($SO_3H$)-Met-Gly-Trp-Met-Asp-MePhe-$NH_2$, wobei Hpa 4-Hydroxyphenylessigsäure bedeutet, oder ein pharmazeutisch annehmbares Salz hievon ist.

**7.** Verbindung nach Anspruch 1, bei welchem die Verbindung der Formel (I)
Hpa($SO_3H$)-Met-Gly-Trp-Met-Asp-Phe-$NH_2$;
Hpa($SO_3H$)-Ala-Gly-Trp-Met-Asp-Phe-$NH_2$;
Hpa($SO_3H$)-Ala-Gly-Trp-Met-Asp-MePhe-$NH_2$;
Hpa($SO_3H$)-Met-DAla-Trp-Met-Asp-MePhe-$NH_2$;
Hpa($SO_3H$)-Met-Gly-Ala-Met-Asp-MePhe-$NH_2$;
Hpa($SO_3H$)-Met-Gly-Trp-Ala-Asp-MePhe-$NH_2$;
Hpa($SO_3H$)-Met-Gly-Trp-Met-Ala-MePhe-$NH_2$;
Hpa($SO_3H$)-Ala-Gly-Trp-Ala-Asp-MePhe-$NH_2$;
Hpa($SO_3H$)-Ahx-Gly-Trp-Ahx-Asp-MePhe-$NH_2$;
Hpa($SO_3H$)-Ile-Gly-Trp-Ile-Asp-MePhe-$NH_2$;
Hpa($SO_3H$)-Ile-Gly-Trp-Ahx-Asp-MePhe-$NH_2$;
Hpa($SO_3H$)-Met-Ala-Trp-Met-Asp-MePhe-$NH_2$;
Hpa($SO_3H$)-Met-$\beta$Ala-Trp-Met-Asp-MePhe-$NH_2$;
Hpa($SO_3H$)-Met-Gly-Trp-Met-DAsp-MePhe-$NH_2$;
oder ein pharmazeutisch annehmbares Salz hievon ist.

**8.** Verfahren zur Verbesserung des körperlichen Aussehens eines Säugers, welches das intraperitoneale, intravenöse, intramuskuläre, subkutane oder intranasale Verabreichen einer Verbindung der Formel (I), wie in Anspruch 1 definiert, oder eines pharmazeutisch annehmbaren Salzes hievon, an diesen Säuger umfaßt, bis ein kosmetisch vorteilhafter Körpergewichtsverlust eingetreten ist.

**Revendications**

**Revendications pour les Etats contractants suivants : AT, BE, CH, DE, DK, FR, GB, IT, LI, LU, NL, SE**

1. Composé de formule (I) :

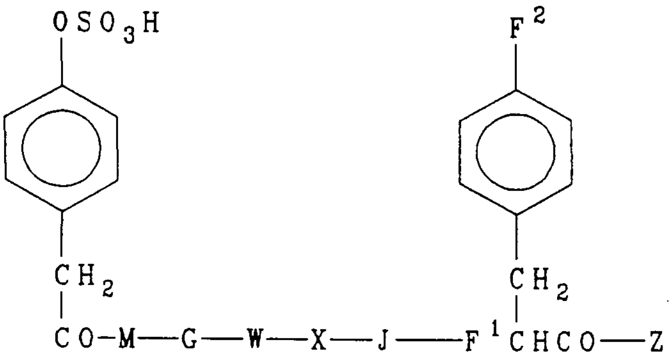

(I)

dans lequel :

M est Met, D-Met, MeMet, MetO, Ahx, D-Ahx, MeAhx, Leu, MeLeu, Pro, Ile, MeIle, Ala ou Lys;
G est Gly, D-Ala, Pro, Ala, $\beta$-Ala ou Sar;
W est Trp, MeTrp, Ala ou Nal;
X est Met, MeMet, MetO, Ahx, MeAhx, Leu, MeLeu, Pro, Ile, MeIle, Ala ou Lys;
J est Asp, D-Asp, MeAsp, Ala ou Asn;
$F^1$ est (S)-NH, (R)-NH, (S)-$R^1$N ou (R)-$R^2$N;
$F^2$ est H, Cl, I, Br, F, $NO_2$, $NH_2$, $R^3$ ou $OR^4$;
Z est $NH_2$, $NHR^5$ ou $NR^5R^6$;
$R^1$, $R^2$, $R^3$,$R^5$ et $R^6$ sont alcoyle en C1 a C6, et
$R^4$ est H ou alcoyle en C1 a C6,
et les sels pharmaceutiquement acceptables de celui-ci, dans lesquels Ahx représente l'acide 2-aminohexanoïque.

2. Composé suivant la revendication 1, dans lequel M est Met, Ahx, Leu, Ala ou Ile.

3. Composé suivant l'une quelconque des revendications précédentes, dans lequel X est Met, Ahx, Leu ou Ile.

4. Composé suivant l'une quelconque des revendications précédentes, dans lequel $F^1$ est (S)-NH ou (S)-$R^2$N.

5. Composé suivant l'une quelconque des revendications précédentes, dans lequel Z est $NH_2$.

6. Composé suivant la revendication 1, dans lequel le composé de formule (I) est :
Hpa($SO_3$H)-Met-Gly-Trp-Met-Asp-MePhe-$NH_2$;
ou un sel pharmaceutiquement acceptable de celui-ci, dans lequel Hpa représente l'acide 4-hydroxyphénylacétique.

7. Composé suivant la revendication 1, dans lequel le composé de formule (I) est :
Hpa($SO_3$H)-Met-Gly-Trp-Met-Asp-Phe-$NH_2$;
Hpa($SO_3$H)-Ala-Gly-Trp-Met-Asp-Phe-$NH_2$;
Hpa($SO_3$H)-Ala-Gly-Trp-Met-Asp-MePhe-$NH_2$;
Hpa($SO_3$H)-Met-D-Ala-Trp-Met-Asp-MePhe-$NH_2$;
Hpa($SO_3$H)-Met-Gly-Ala-Met-Asp-MePhe-$NH_2$;
Hpa($SO_3$H)-Met-Gly-Trp-Ala-Asp-MePhe-$NH_2$;
Hpa($SO_3$H)-Met-Gly-Trp-Met-Ala-MePhe-$NH_2$;
Hpa($SO_3$H)-Ala-Gly-Trp-Ala-Asp-MePhe-$NH_2$;
Hpa($SO_3$H)-Ahx-Gly-Trp-Ahx-Asp-MePhe-$NH_2$;

Hpa($SO_3$H)-Ile-Gly-Trp-Ile-Asp-MePhe-$NH_2$;
Hpa($SO_3$H)-Ile-Gly-Trp-Ahx-Asp-MePhe-$NH_2$;
Hpa($SO_3$H)-Met-Ala-Trp-Met-Asp-MePhe-$NH_2$;
Hpa($SO_3$H)-Met-$\beta$-Ala-Trp-Met-Asp-MePhe-$NH_2$;
Hpa($SO_3$H)-Met-Gly-Trp-Met-D-Asp-MePhe-$NH_2$;
ou un sel pharmaceutiquement acceptable de ceux-ci.

**8.** Composition pharmaceutique comprenant un composé de formule (I) tel que défini à la revendication 1, ou un sel pharmaceutiquement acceptable de celui-ci, en mélange avec un support pharmaceutiquement acceptable.

**9.** Utilisation d'un composé de formule (I) tel que défini à la revendication 1, ou d'un sel pharmaceutiquement acceptable de celui-ci, dans la préparation d'un médicament pour le traitement ou la prévention de l'obésité.

**10.** Procédé de préparation de composés de formule (I) tels que défini à la revendication 1, qui comprend :
a) la sulfonation d'un composé de formule (II) :

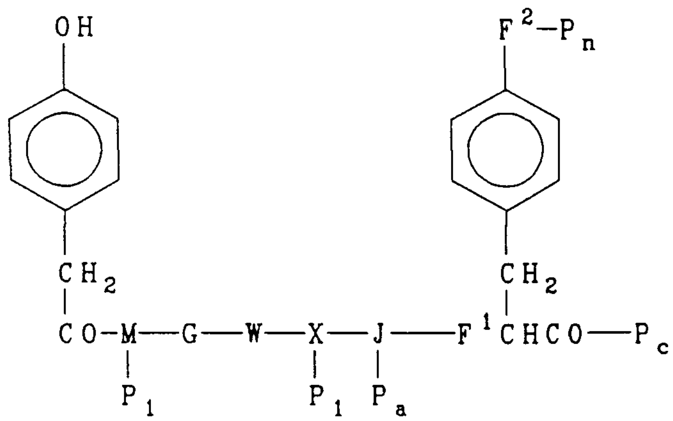

(II)

dans lequel :
M, G, W, X, J, $F^1$, $F^2$ et Z sont tels que définis ci-dessus;
$P_1$ représente un radical protecteur d'amino lorsque M ou X représente Lys;
$P_a$ représente un radical protecteur de carboxyle lorsque J représente Asp, D-Asp ou MeAsp;
$P_n$ représente un radical protecteur d'hydroxyle ou un radical protecteur d'amino lorsque $F^2$ représente $NH_2$ ou OH, et
$P_c$ représente Z ou un radical protecteur de carboxyle;
b) l'elimination d'un ou de plusieurs groupes protecteurs d'un composé correspondant de formule (III) :

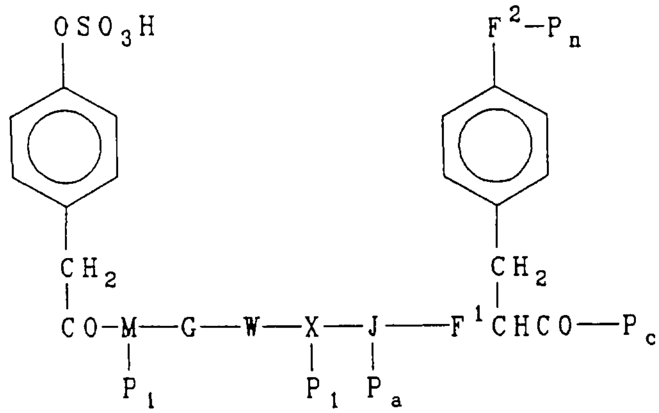

(III)

dans lequel :

M, G, W, X, J, $F^1$, $F^2$, $P_1$, $P_a$, $P_n$ et $P_c$ sont tels que définis ci-dessus,

pourvu qu'au moins un des $P_1$, $P_a$, $P_n$ et $P_c$ représente un radical protecteur, et, si désiré ou nécessaire, la conversion du composé résultant de formule (I) en un sel pharmaceutiquement acceptable de celui-ci ou vice versa.

**11.** Composé de formule (I) tel que défini à la revendication 1, ou un sel pharmaceutiquement acceptable de celui-ci, à utiliser comme produit pharmaceutique.

**12.** Procédé d'amélioration de l'apparence corporelle d'un mammifère, qui comprend l'administration à un mammifère, de manière intrapéritonéale, intraveineuse, intramusculaire, sous-cutanée ou intranasale, d'un composé de formule (I) tel que défini à la revendication 1, ou d'un sel pharmaceutiquement acceptable de celui-ci, jusqu'à ce qu'une perte de poids esthétiquement bénéfique se produise.

**Revendications pour les Etats contractants suivants : ES, GR**

**1.** Procédé de préparation d'un composé de formule (I) :

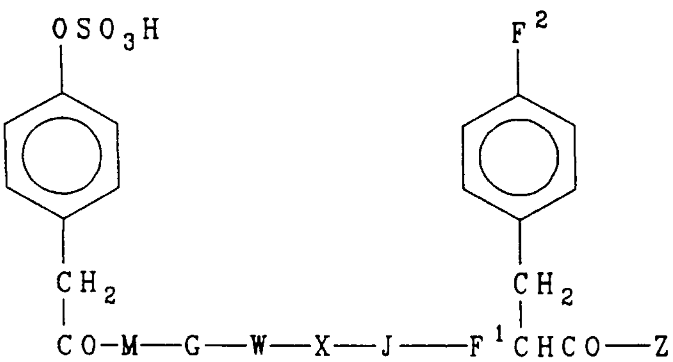

(I)

dans lequel :

M est Met, D-Met, MeMet, MetO, Ahx, D-Ahx, MeAhx, Leu, MeLeu, Pro, Ile, MeIle, Ala ou Lys;

G est Gly, D-Ala, Pro, $\beta$-Ala, Ala ou Sar;

W est Trp, MeTrp, Ala ou Nal;

X est Met, MeMet, MetO, Ahx, MeAhx, Leu, MeLeu, Pro, Ile, MeIle, Ala ou Lys;

J est Asp, D-Asp, MeAsp, Ala ou Asn;

$F^1$ est (S)-NH, (R)-NH, (S)-$R^1$N ou (R)-$R^2$N;

$F^2$ est H, Cl, I, Br, F, $NO_2$, $NH_2$, $R^3$ ou $OR^4$;

Z est $NH_2$, $NHR^5$ ou $NR^5R^6$;

$R^1$, $R^2$, $R^3$,$R^5$ et $R^6$ sont alcoyle en C1 à C6, et

$R^4$ est H ou alcoyle en C1 à C6,

dans lequel Ahx représente l'acide 2-aminohexanoïque, ou d'un sel pharmaceutiquement acceptable de celui-ci, qui comprend :

a) la sulfonation d'un composé de formule (II) :

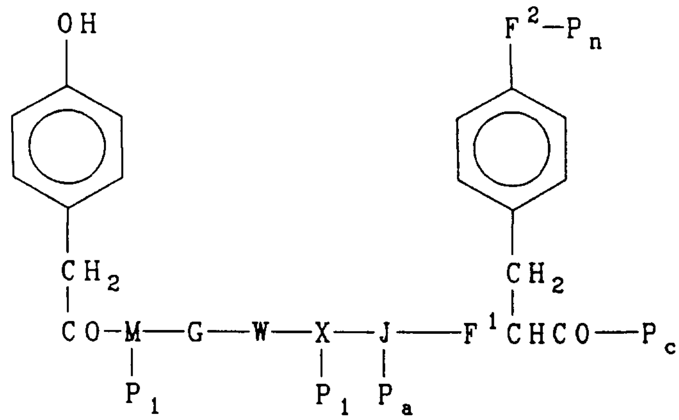

(II)

dans lequel :

M, G, W, X, J, $F^1$, $F^2$ et Z sont tels que définis ci-dessus;

$P_1$ représente un radical protecteur d'amino lorsque M ou X représente Lys;

$P_a$ représente un radical protecteur de carboxyle lorsque J représente Asp, D-Asp ou MeAsp;

$P_n$ représente un radical protecteur d'hydroxyle ou un radical protecteur d'amino lorsque $F^2$ représente $NH_2$ ou OH, et

$P_c$ représente Z ou un radical protecteur de carboxyle;

b) l'elimination d'un ou de plusieurs groupes protecteurs d'un composé correspondant de formule (III) :

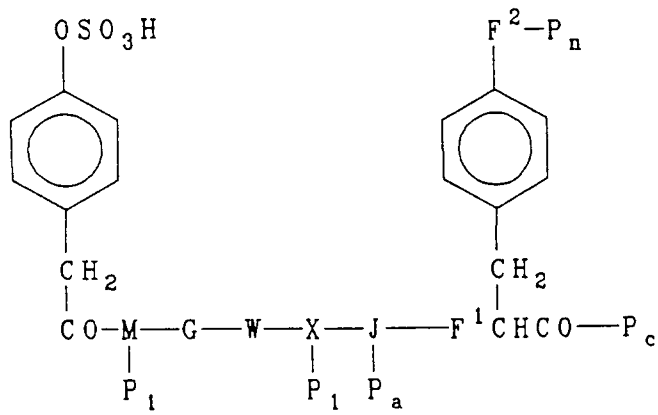

(III)

dans lequel :

M, G, W, X, J, $F^1$, $F^2$, $P_1$, $P_a$, $P_n$ et $P_c$ sont tels que définis ci-dessus,

pourvu qu'au moins un des $P_1$, $P_a$, $P_n$ et $P_c$ représente un radical protecteur, et, si désiré ou nécessaire, la conversion du composé résultant de formule (I) en un sel pharmaceutiquement acceptable de celui-ci ou vice versa.

**2.** Procédé suivant la revendication 1, dans lequel M est Met, Ahx, Leu, Ala ou Ile.

**3.** Procédé suivant l'une quelconque des revendications précédentes, dans lequel X est Met, Ahx, Leu, ou Ile.

**4.** Procédé suivant l'une quelconque des revendications précédentes, dans lequel $F^1$ est (S)-NH ou (S)-$R^2N$.

**5.** Procédé suivant l'une quelconque des revendications précédentes, dans lequel Z est $NH_2$.

6. Procédé suivant la revendication 1, dans lequel le composé de formule (I) est :
   $Hpa(SO_3H)$-Met-Gly-Trp-Met-Asp-MePhe-$NH_2$;
   dans lequel Hpa représente l'acide 4-hydroxyphénylacétique ou un sel pharmaceutiquement acceptable de celui-ci.

7. Procédé suivant la revendication 1, dans lequel le composé de formule (I) est :
   $Hpa(SO_3H)$-Met-Gly-Trp-Met-Asp-Phe-$NH_2$;
   $Hpa(SO_3H)$-Ala-Gly-Trp-Met-Asp-Phe-$NH_2$;
   $Hpa(SO_3H)$-Ala-Gly-Trp-Met-Asp-MePhe-$NH_2$;
   $Hpa(SO_3H)$-Met-D-Ala-Trp-Met-Asp-MePhe-$NH_2$;
   $Hpa(SO_3H)$-Met-Gly-Ala-Met-Asp-MePhe-$NH_2$;
   $Hpa(SO_3H)$-Met-Gly-Trp-Ala-Asp-MePhe-$NH_2$;
   $Hpa(SO_3H)$-Met-Gly-Trp-Met-Ala-MePhe-$NH_2$;
   $Hpa(SO_3H)$-Ala-Gly-Trp-Ala-Asp-MePhe-$NH_2$;
   $Hpa(SO_3H)$-Ahx-Gly-Trp-Ahx-Asp-MePhe-$NH_2$;
   $Hpa(SO_3H)$-Ile-Gly-Trp-Ile-Asp-MePhe-$NH_2$;
   $Hpa(SO_3H)$-Ile-Gly-Trp-Ahx-Asp-MePhe-$NH_2$;
   $Hpa(SO_3H)$-Met-Ala-Trp-Met-Asp-MePhe-$NH_2$;
   $Hpa(SO_3H)$-Met-$\beta$-Ala-Trp-Met-Asp-MePhe-$NH_2$;
   $Hpa(SO_3H)$-Met-Gly-Trp-Met-D-Asp-MePhe-$NH_2$;
   ou un sel pharmaceutiquement acceptable de ceux-ci.

8. Procédé d'amélioration de l'apparence corporelle d'un mammifère, qui comprend l'administration à un mammifère, de manière intrapéritonéale, intraveineuse, intramusculaire, sous-cutanée ou intranasale, d'un composé de formule (I) tel que défini à la revendication 1, ou d'un sel pharmaceutiquement acceptable de celui-ci, jusqu'à ce qu'une perte de poids esthétiquement bénéfique se produise.